(19) Europäisches Patentamt
European Patent Office
Office européen des brevets

(11) Publication number: 0 146 333 A1

# (12) EUROPEAN PATENT APPLICATION

(21) Application number: 84308609.1

(22) Date of filing: 11.12.84

(51) Int. Cl.⁴: **C 07 D 257/04**, C 07 C 121/75

(30) Priority: 16.12.83 GB 8333666

(43) Date of publication of application: 26.06.85 **Bulletin 85/26**

(84) Designated Contracting States: **AT BE CH DE FR GB IT LI LU NL SE**

(71) Applicant: **Lilly Industries Limited, Lilly House Hanover Square, London W1R 0PA (GB)**

(72) Inventor: **Goldsworthy, John, 4 Tawfield, Great Hollands, Bracknell Berkshire (GB)**
Inventor: **Verge, John Pomfret, 2 Chestnut Close Middle Assendon, Henley-on-Thames Oxfordshire (GB)**

(74) Representative: **Hudson, Christopher Mark et al, Erl Wood Manor, Windlesham Surrey GU20 6PH (GB)**

## (54) Organic compounds and their pharmaceutical use.

(57) There is described compounds of the formula

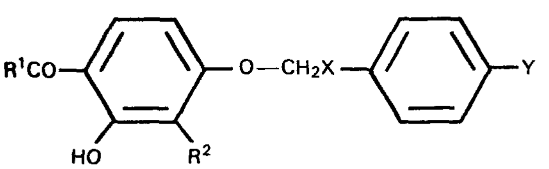
(I)

in which $R^1$ is hydrogen or $C_{1-6}$ alkyl, $R^2$ is hydrogen, $C_{1-6}$ alkyl or $C_{3-6}$ alkenyl, and X is $-CH=CH-$ or $-(CH_2)_n-$ where n is 1 to 3, and Y is -CN or

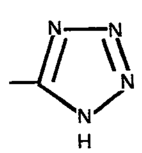
;

and salts thereof. The compounds in which Y is tetrazolyl possess pharmaceutical activity.

EP 0 146 333 A1

0146333

G.1219

# ORGANIC COMPOUNDS AND THEIR PHARMACEUTICAL USE

This invention relates to novel compounds, pharmaceutical compositions containing them and their use as pharmaceuticals.

The compounds of the invention have the formula

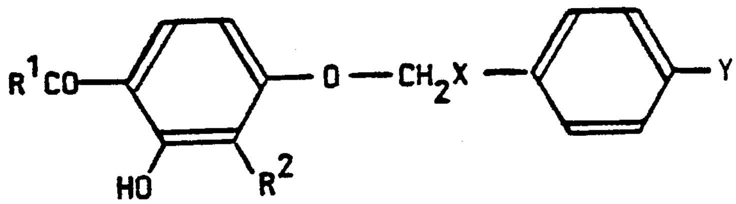

(I)

in which $R^1$ is hydrogen or $C_{1-6}$ alkyl, $R^2$ is hydrogen, $C_{1-6}$ alkyl or $C_{3-6}$ alkenyl, X is -CH=CH- or $-(CH_2)_n-$ where n is 1 to 3, and Y is -CN or

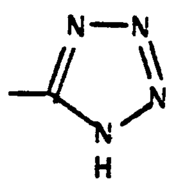

;

and salts thereof.

Compounds of the above formula (I), with the exception of those in which Y is -CN which are intermediates in the

preparation of the remaining compounds, are inhibitors of leukotriene action, and are thus indicated for use in a variety of pharmacological conditions. For example, they may be used in the prophylactic and therapeutic treatment of immediate hypersensitivity diseases including asthma and in the alleviation of status asthmaticus.

In the above formula (I) reference to a "$C_{1-6}$ alkyl" group includes, for example, methyl, ethyl, propyl, isopropyl and tert. butyl, and is preferably methyl, ethyl or propyl. A "$C_{3-6}$ alkenyl" group includes, for example, allyl, isopropenyl, butenyl, isobutenyl and 3-methyl-2-butenyl, and is preferably allyl.

Preferred groups of compounds of formula (I) are those in which $R^1$ is $C_{1-4}$ alkyl, $R^2$ is $C_{1-4}$ alkyl and X is $-CH=CH-$ or $-(CH_2)_n-$ where n is 2 or 3.

It will be appreciated that compounds in which X is $-CH=CH-$ exist in stereoisomeric (Z and E) forms such as follows

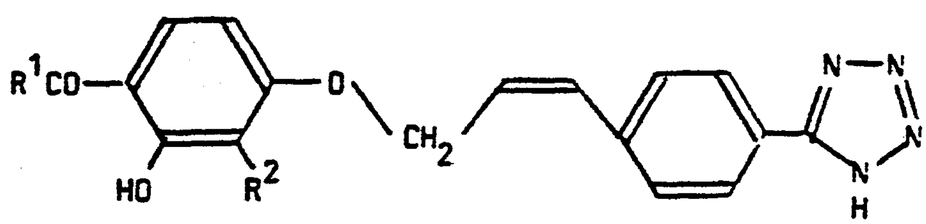

and

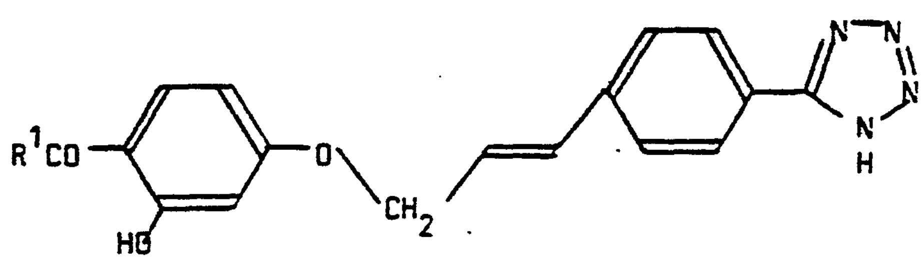

Since the active compounds of formula (I) bear a tetrazolyl group, an opportunity exists of forming base addition salts, and such salts are included as part of the present invention. Examples of such salts are those derived from ammonium hydroxide and alkali and alkaline earth metal hydroxides, carbonates and bicarbonates, as well as salts derived from aliphatic and aromatic amines, aliphatic diamines and hydroxy alkylamines. Bases especially useful in the preparation of such salts include ammonium hydroxide, potassium carbonate, sodium bicarbonate, calcium hydroxide, methylamine, diethylamine, ethylene diamine, cyclohexylamine and ethanolamine. The potassium and sodium salt forms are particularly preferred.

Apart from pharmaceutically acceptable addition salts, other salts are also included within the scope of the invention since they may serve as intermediates in the purification of compounds or in the preparation of other pharmaceutically acceptable salts, or they may be useful for identification, characterization or purification of the free compound.

The pharmaceutical compounds of the invention may be prepared by reacting the intermediate of the formula

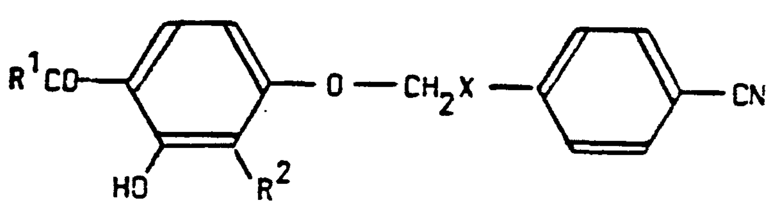

(II)

where $R^1$, $R^2$ and X have the values given above, with an azide. Compounds of formula (II) are novel and form part of the present invention.

The process is preferably carried out in an organic solvent such as a polar aprotic solvent, for example dimethylformamide, and suitably at a temperature in the range of from 20°C to 150°C, for example, from 90°C to 120°C. The azide employed can be, for example, an alkali metal azide such as sodium azide, and we have found that a combination of alkali metal azide and ammonium chloride is especially convenient.

As mentioned above, if it is desired to produce the E-isomer of the compound in which X is -CH=CH- the Z-isomer prepared by reaction of azide with nitrile compound of formula (II) can be irradiated using intense light, optionally with for example diphenyl disulphide as radical source. Alternatively, the Z-isomer is prepared from the appropriate compound of formula (II) with Z configuration.

Compounds of formula (II) may be prepared by condensing an appropriate phenol reactant of the formula

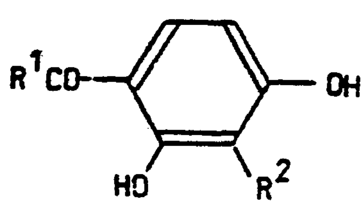

(III)

with a nitrile intermediate of the formula

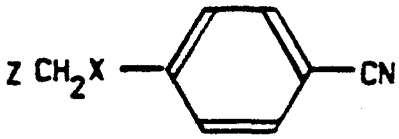

(IV)

where Z is halo, especially bromo, in a suitable organic solvent and in the presence of an alkali metal hydride such as for example sodium hydride.

In their turn, compounds of formula (IV) can be prepared from derivatives of the formula

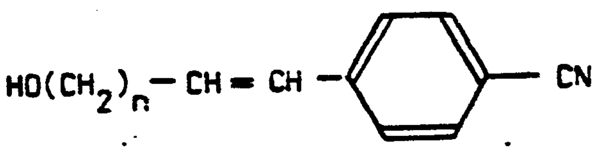

where n is 1, 2 or 3, which are prepared by reaction of 4-cyanobenzaldehyde with the appropriate triphenylphosphonium bromide derivative, with optional subsequent reduction.

The compounds of the present invention are pharmacologically active, being inhibitors of leukotriene action as shown by the following tests: (a) the *in vitro* test on guinea pig ileum segments at concentrations of from 10 ng to 50 μg, according to the method of Schild, 1947 Brit. J. Pharm. 2 197-206, using $LTD_4$ as the antagonist (the pharmacological compounds of the following Examples exhibited an $IC_{50}$ against

$LTD_4$ of less than $10^{-4}$ molar); (b) the *in vivo* Guinea Pig Pulmonary Function Test of Austen and Drazen 1974 J. Clin. Invest. 53 1679-1685 at intravenous dosage levels of from 0.05 μg to 5.0 mg/kg; and (c) a modified "Herxheimer" test, Journal of Physiology London 117 251 (1952), at doses of from 25 to 200 mg/kg. The "Herxheimer" test is based on an $LTD_4$-induced bronchospasm in guinea pigs which closely resembles an asthmatic attack in man. The compounds also inhibit the formation of leukotrienes, as indicated by their action in the test described by Harvey and Osborne, Journal of Pharmacological Methods 9 147-155 (1983).

The compounds are accordingly indicated for therapeutic use in the treatment of diseases in which leukotrienes are implicated. These include immediate hypersensitivity diseases, allergic reactions of the pulmonary system in which leukotrienes are thought to be causal mediators of bronchospasm, for example, in allergic lung disorders such as extrinsic asthma and industrial asthmas such as Farmers lung and Pigeon Fanciers lung, and in other inflammatory disorders, for example, associated with acute or chronic infectious diseases such as allergic skin diseases, ectopic and atopic eczemas, psoriasis, contact hypersensitivity and angioneurotic oedema, bronchitis and cystic fibrosis and rheumatic fever.

The compounds may be administered by various routes, for example, by the oral or rectal route, by inhalation, topically or parenterally, for example by injection, being usually employed in the form of a pharmaceutical composition. Such compositions form part of the present invention and are prepared in a manner well known in the pharmaceutical art and

normally comprise at least one active compound in association with a pharmaceutically acceptable diluent or carrier. In making the compositions of the present invention, the active ingredient will usually be mixed with a carrier, or diluted by a carrier, and/or enclosed within a carrier which may, for example, be in the form of a capsule, sachet, paper or other container. Where the carrier serves as a diluent, it may be a solid, semi-solid, or liquid material which acts as a vehicle, excipient or medium for the active ingredient. Thus, the composition may be in the form of tablets, lozenges, sachets, cachets, elixirs, suspensions, aerosols as a solid or in a liquid medium, ointments containing for example up to 10% by weight of the active compound, soft and hard gelatin capsules, suppositories, injection solutions and suspensions and sterile packaged powders. For administration by inhalation, particular forms of presentation include aerosols, atomisers and vaporisers.

Some examples of suitable carriers are lactose, dextrose, sucrose, sorbitol, mannitol, starches, gum acacia, calcium phosphate, alginates, tragacanth, gelatin, syrup, methyl cellulose, methyl- and propyl- hydroxybenzoate, talc, magnesium stearate and mineral oil. The compositions of the invention may, as is well known in the art, be formulated so as to provide quick, sustained or delayed release of the active ingredient after administration to the patient.

Where the compositions are formulated in unit dosage form, it is preferred that each unit dosage form contains from 5 mg to 500 mg, more usually 25 to 200 mg, of the active ingredient. The term "unit dosage form" refers to physically discrete units suitable as unit dosages for human subjects and

animals, each unit containing a predetermined quantity of active material calculated to produce the desired therapeutic effect, in association with the required pharmaceutical carrier.

The active compounds are effective over a wide dosage range and for example dosages per day will normally fall within the range of 0.5 to 300 mg/kg. and in the treatment of adult humans, more usually in the range of from 5 to 100 mg/kg. However it will be understood that the amount of the compound actually administered will be determined by a physician, in the light of the relevant circumstances including the condition to be treated, the choice of compound to be administered and the chosen route of administration and therefore the above dosage ranges are not intended to limit the scope of the invention in any way.

The following Examples illustrate the invention.

EXAMPLE 1

(E,Z)-1-(Methoxy-dimethyl-methoxy)-3-(4-cyanophenyl)-prop-2-ene

To a suspension of 2-(methoxy-dimethyl-methoxy)-ethyltriphenylphosphonium bromide (prepared by the method of E.J. Corey et al. J.A.C.S. 102, 7986 (1980), from 2-hydroxyethyl-triphenylphosphonium bromide and 2-methoxypropene) (178.8 g) in dry tetrahydrofuran (700 ml), under nitrogen, at -70°C, was added n-butyl lithium (266 ml of 1.55 M solution in hexane), by cannula from a measuring cylinder under nitrogen pressure. The generation of the ylid was shown by the development of a deep orange colour. The mixture was allowed to warm to -30°C

and then re-cooled. A solution of 4-cyano-benzaldehyde (51.03 g) in dry tetrahydrofuran (200 ml) was added, and the mixture allowed to attain room temperature. The orange colour was quenched to a straw yellow. The reaction mixture was stirred at room temperature for 3 hours then filtered over High-Flo. The filtrates were evaporated in vacuo and the residual oil extracted with ether (8 x 250 ml). Evaporation of the extracts gave the crude product as an oil. This was further purified by column chromatography (silica, eluant:hexane 50% diethyl ether) to give the product as a yellow oil, consisting of Z:E = 3:1 (Fourier Transform PMR analysis).

EXAMPLE 2

(E,Z)-3-(4-Cyanophenyl)-prop-2-ene-1-ol

(E,Z)-1-(Methoxydimethylmethoxy)-3-(4-cyanophenyl)-prop-2-ene (12.1 g) was dissolved in a mixture of glacial acetic acid (1.5 ml), acetonitrile (96 ml) and water (24 ml) and the solution allowed to stand at room temperature under nitrogen for 60 hours.

The reaction mixture was neutralised with triethylamine (8 ml) and evaporated in vacuo to a yellow oil. The oil was partitioned between water (100 ml) and dichloromethane (2 x 100 ml), the combined organic extracts washed with water (2 x 200 ml), dried ($MgSO_4$) and evaporated to yield the product as a yellow oil.

EXAMPLE 3

(E,Z)-1-Bromo-3-(4-cyanophenyl)-prop-2-ene

To a stirred solution of (E,Z)-3-(4-cyanophenyl)-prop-2-ene-1-ol (3.60 g) in dichloromethane (15 ml) at -5°C under nitrogen was added dropwise a solution of phosphorus tribromide (0.7 ml) in dichloromethane (5 ml). The reaction mixture was then allowed to stand at room temperature for 24 hours, then washed with brine (2 x 20 ml), dried ($MgSO_4$) and evaporated to yield the product as a yellow oil.

EXAMPLE 4

(E,Z)-4-[3-(4-Acetyl-3-hydroxy-2-propylphenoxy)-1-propenyl]benzonitrile

A mixture of 2,4-dihydroxy-3-propylacetophenone (1.84 g), sodium carbonate (3.01 g) and (E,Z)-1-bromo-3-(4-cyanophenyl)-prop-2-ene (2.10 g) in dry butanone (40 ml) was refluxed for 24 hours. The reaction mixture was filtered, the filtrates evaporated *in vacuo*, and the residue partitioned between water (50 ml) and dichloromethane (2 x 50 ml). The combined organic extracts were washed with 2 molar sodium hydroxide (2 x 100 ml) and then water (2 x 100 ml), dried ($MgSO_4$) and evaporated to give an amber oil which was a mixture of the E and Z forms of the composition indicated in Example 1. The oil was then purified by column chromatography (silica, eluent:hexane 40% ethyl acetate) to yield the separated geometrical isomers:-

(i) Z isomer, yellow solid, m.p. 104-106°C.

(ii) E isomer, yellow solid, m.p. 116-118°C.

EXAMPLE 5

(Z)-1-{2-Hydroxy-3-propyl-4-[3-(4-1H-tetrazol-5-yl-phenyl)-prop-2-enyloxy]-phenyl}ethanone

A mixture of (Z)-4-[3-(4-acetyl-3-hydroxy-2-propylphenoxy)-1-propenyl]-benzonitrile (from Example 4) (1.14 g), sodium azide (1.11 g) and ammonium chloride (0.90 g) in dry dimethylformamide (10 ml) was stirred at 100°C for 4 hours. The reaction mixture was cooled, poured into water (250 ml) and stirred vigorously for 15 minutes. The suspended solid was then collected by filtration and dissolved in 2 molar sodium hydroxide solution (50 ml). The basic solution was washed with diethyl ether (2 x 50 ml), filtered and acidified with concentrated hydrochloric acid. The precipitated solid was collected by filtration, washed with water on the sinter, and dried in vacuo. Finally recrystallisation from ethanol/water with decolourisation gave the title compound as a fawn solid, m.p. 162-166°C.

EXAMPLE 6

(E)-1-{2-Hydroxy-3-propyl-4-[3-(4-1H-tetrazol-5-yl-phenyl)-prop-2-enyloxy]-phenyl}ethanone

A mixture of (E)-4-[3-(4-acetyl-3-hydroxy-2-propylphenoxy)-1-propenyl]-benzonitrile (from Example 4) (2.32 g), sodium azide (2.25 g) and ammonium chloride (1.85 g) in dry dimethylformamide (25 ml) was stirred at 100°C for 4 hours. The reaction mixture was allowed to cool, then stirred with water (1 litre) for 10 minutes. The suspended solid was filtered off, and dissolved in a 2 molar solution of sodium hydroxide (200 ml). The basic solution was washed with

diethyl ether (2 x 200 ml), filtered, and acidified with concentrated hydrochloric acid. The precipitated solid was collected by filtration, washed with water on the sinter, and dried *in vacuo*. Recrystallisation from ethanol/water with decolourisation gave the title compound as a fawn solid, m.p. 186-190°C.

EXAMPLE 7

(E)-1-{2-Hydroxy-3-propyl-4-[3-(4-1H-tetrazol-5-ylphenyl)-prop-2-enyloxy]-phenyl}ethanone

(Alternative procedure to Example 6)

A mixture of (Z)-1-{2-hydroxy-3-propyl-4-[3-(4-1H-tetrazol-5-yl-phenyl)-prop-2-enyloxy]-phenyl}ethanone (100 mg) and diphenyldisulphide (10 mg) in methanol (5 ml) was stirred and irradiated under a quartz lamp for 24 hours. The reaction mixture was evaporated *in vacuo* and excess diphenyl disulphide removed from the residue by chromatography (silica, eluent: chloroform 5% methanol). Final recrystallisation from ethanol/water with decolourisation gave the title compound as an off-white solid, m.p. 182-186°C.

EXAMPLE 8

3-(4-Cyanophenyl)-propan-1-ol

A solution of (E,Z)-3-(4-cyanophenyl)-prop-2-ene-1-ol (from Example 2) (3.60 g) in absolute ethanol (100 ml) was hydrogenated at room temperature and 60 p.s.i. using 10% palladium on charcoal (100 mg) as catalyst. Hydrogen uptake was complete in a few minutes. The reaction mixture was filtered over High-Flo, and the catalyst cake washed with

chloroform. The combined filtrates and washings were evaporated *in vacuo* to give the product as a light-yellow oil.

EXAMPLE 9

1-Bromo-3-(4-cyanophenyl)-propane

To a stirred solution of 3-(4-cyanophenyl)-propan-1-ol (2.80 g) in dichloromethane (15 ml) at -5°C under nitrogen was added dropwise a solution of phosphorus tribromide (0.55 ml) in dichloromethane (5 ml). The reaction mixture was then allowed to stand at room temperature for 24 hours, washed with brine (2 x 20 ml), dried ($MgSO_4$) and evaporated *in vacuo* to give a yellow oil. The oil was distilled in a bulb-to-bulb apparatus to give the product as a clear oil, b.p. 120°C/0.35 mmHg.

EXAMPLE 10

4-[3-(4-Acetyl-3-hydroxy-2-propylphenoxy)-propyl]-benzonitrile

To a suspension of washed (toluene 3x) 50% sodium hydride (0.11 g) in dry dimethylformamide (5 ml) under nitrogen was added a solution of 2,4-dihydroxy-3-propyl-actophenone (0.87 g) in dry dimethylformamide (5 ml). The mixture was then heated at 60°C for 15 minutes, and a solution of 1-bromo-3-(4-cyanophenyl)-propane (1.00 g) in dry dimethylformamide (5 ml) was added dropwise. Stirring was continued at 100°C for 4 hours, and then the reaction mixture was evaporated *in vacuo*. The residual oil was stirred with water (50 ml) and extracted with dichloromethane (2 x 50 ml); the combined extracts washed with 2 M sodium hydroxide (2 x 100 ml) and water (2 x 100 ml), dried ($MgSO_4$) and evaporated *in vacuo* to give the product as a brown oil.

EXAMPLE 11

1-{2-Hydroxy-3-propyl-4-[3-(4-1H-tetrazol-5-yl-phenyl)-propoxy]-phenyl}ethanone

A mixture of 4-[3-(4-acetyl-3-hydroxy-2-propylphenoxy)-propyl]-benzonitrile (1.18 g) sodium azide (0.91 g) and ammonium chloride (0.75 g) in dry dimethylformamide (20 ml) was stirred at 100°C for 4 hours. The reaction mixture was allowed to cool, poured into water (600 ml) and stirred for 10 minutes. The suspended solid was collected by filtration, dried *in vacuo* and recrystallised from ethanol/water with decolourisation to give the title compound as a fawn solid, m.p. 190-192°C.

Similarly prepared, utilizing the procedures of Examples 1, 2, 8, 9, 10 and 11 was:-

1-{2-Hydroxy-3-propyl-4-[4-(4-1H-tetrazol-5-yl-phenyl)-butoxy]-phenyl}ethanone, m.p. 150-154°C.

The following Examples illustrate the preparation of typical formulations comprising a pharmacologically active compound of the invention.

EXAMPLE 12

Aerosol

| | |
|---|---|
| Active ingredient | 100 mg |
| Ethanol | 30 ml |
| Propellent 12/114 | q.s. |

The active ingredient is dissolved in ethanol, filled into glass bottles, sealed with a valve (metered to 0.05 ml) and charged with the mixed propellants.

EXAMPLE 13

Tablet

| | |
|---|---|
| Active ingredient | 100 mg |
| Dried starch | 400 mg |
| Polyvinyl pyrrolidone | 50 mg |
| Sodium carboxymethyl starch | 50 mg |
| Stearic acid | 20 mg |

The active ingredient and starch are mixed together and massed with a solution of polyvinyl pyrrolidone in alcohol. The mass is extruded through a screen, dried, sized and mixed with sodium carboxymethyl starch and stearic acid prior to compression on a tablet machine. Tablets weighing 620 mg are obtained.

EXAMPLE 14

Capsules

| | |
|---|---|
| Active ingredient | 50 mg |
| Starch flowable | 300 mg |
| Silicone fluid | 5 mg |

A portion of the starch is mixed with the silicone fluid. To the powder is added the active ingredient and the remainder of the starch. This blended mixture is filled into hard gelatin capsules.

CLAIMS

1. A compound of the formula

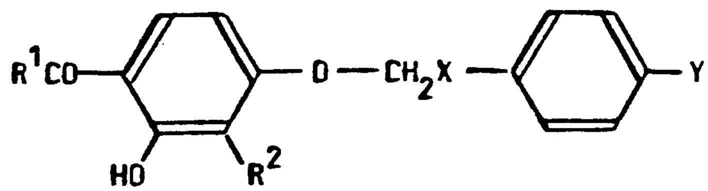 (I)

in which $R^1$ is hydrogen or $C_{1-6}$ alkyl, $R^2$ is hydrogen, $C_{1-6}$ alkyl or $C_{3-6}$ alkenyl, and X is -CH=CH- or $-(CH_2)_n-$ where n is 1 to 3, and Y is -CN or

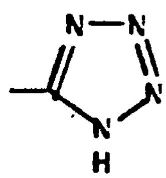 ;

and salts thereof.

2. A compound according to claim 1 in which Y is

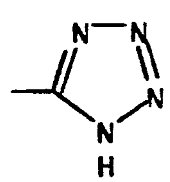

3. A compound according to claim 2 in which $R^1$ is $C_{1-4}$ alkyl, $R^2$ is $C_{1-4}$ alkyl and X is -CH=CH- or $-(CH_2)_n-$ where n is 2 or 3.

4. A compound according to claim 3 in which $R^1$ is methyl and $R^2$ is propyl.

5. A compound according to any of claims 2 to 4 in which X is -CH=CH-.

6. A pharmaceutical formulation comprising a compound as defined in any of claims 2 to 5 or a pharmaceutically-acceptable salt thereof, and a diluent or carrier therefor.

7. A process for preparing a compound according to claim 1 of the formula

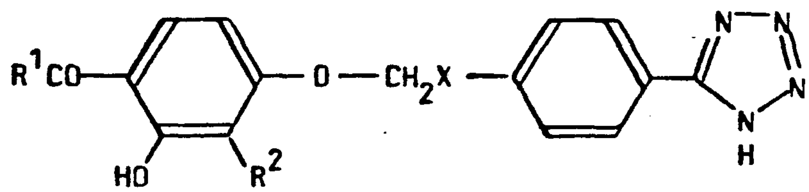

which comprises reacting a compound of the formula

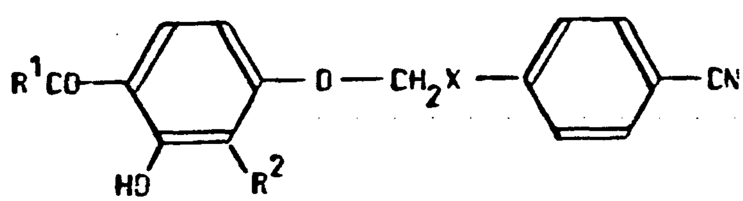

(II)

where $R^1$, $R^2$ and X have the values defined in claim 1, with an azide.

8. A compound of formula (I) as defined in claim 2, for use as a pharmaceutical.

AUSTRIA

(C)

## CLAIMS

1. A process for producing a compound of the formula

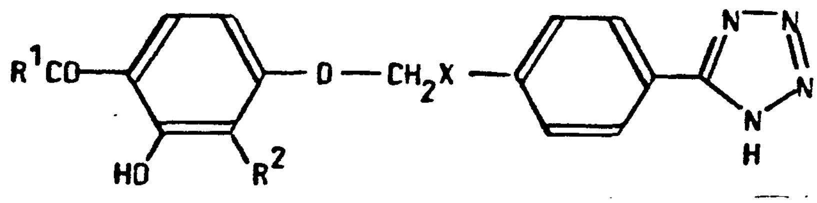

in which $R^1$ is hydrogen or $C_{1-6}$ alkyl, $R^2$ is hydrogen, $C_{1-6}$ alkyl or $C_{3-6}$ alkenyl, and X is -CH=CH- or $-(CH_2)_n-$ where n is 1 to 3, and salts thereof; which comprises reacting a compound of the formula

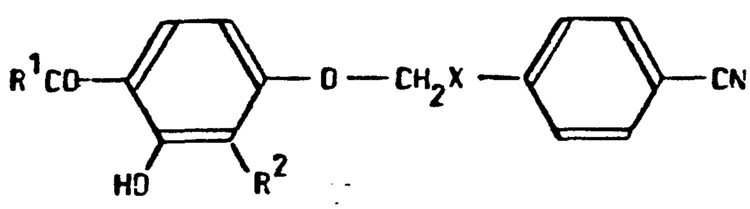

with an azide.

2. A process according to claim 1 for producing a compound in which $R^1$ is $C_{1-4}$ alkyl, $R^2$ is $C_{1-4}$ alkyl and X is -CH=CH- or $-(CH_2)_n-$ where n is 2 or 3.

3. A process according to claim 2 for producing a compound in which $R^1$ is methyl and $R^2$ is propyl.

(C)

4. A process according to claim 3 for producing a compound in which X is -CH=CH-.

0146333

European Patent Office

# EUROPEAN SEARCH REPORT

Application number

EP 84 30 8609

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-2 625 838 (ICI) * Page 21, claim 1 * | 1 | C 07 D 257/04 C 07 C 121/75 |
| A | HELVETICA CHIMICA ACTA, vol. 55, no. 5, 1972, pages 1625-1674, Verlag Helvetica Chimica Acta, Bale, CH; E. SCHMID et al.: "Eine neue 2-Allylphenol-Cumaran-Umlagerung" * Page 1662 sub 7.1.8 * | 1 | |
| A | JUSTUS LIEBIGS ANNALEN DER CHEMIE, vol. 10, 1975, pages 1733-43; G. ZAHL et al.: "Kreuzbeziehungen von Substituenteneinflüssen bei der Claisen-Umlagerung" * Pages 1733-1734, formula 1 * | 1 | |
| | | | TECHNICAL FIELDS SEARCHED (Int. Cl.4) |
| | | | C 07 D 257/00 C 07 C 121/00 |

The present search report has been drawn up for all claims

| Place of search | Date of completion of the search | Examiner |
|---|---|---|
| THE HAGUE | 08-03-1985 | LUYTEN H. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82